# EUROPEAN PATENT SPECIFICATION

(11) **EP 4 436 637 B1**
(45) Date of publication and mention of the grant of the patent: **11.03.2026**
(21) Application number: 22826375.2
(22) Date of filing: 22.11.2022
(51) Int. Cl.: A61M 5/32, A61M 25/00

(54) **MEDICAL CANNULAS**
MEDIZINISCHE KANÜLEN
CANULES MÉDICALES

(30) Priority: 22.11.2021 GB 202116815
(43) Date of publication of application: 02.10.2024
(73) Proprietor: Buckingham Medical Technologies Ltd., Essex CO9 3PY (GB)
(72) Inventor: BUCKINGHAM, Marc, Hedingham Essex CO9 3PY (GB)
(74) Representative: London IP Ltd
(86) International application number: PCT/GB2022/052958
(87) International publication number: WO 2023/089344

(56) References cited:
- US-A- 5 389 079
- US-A1- 2012 078 159
- US-A1- 2013 289 532
- US-A1- 2017 172 653
- US-A1- 2019 167 888

## Description

The present specification relates to medical cannulas, particularly but not exclusively spinal cannulas.

Medical cannulas, which may also be referred to as catheters and needles, are often used to inject substances into, and aspirate (remove) substances from human and animal bodies. One type of cannula often used for spinal injection and aspiration is generally tubular, the end of the tube narrowing to a closed tip, the tip having a blunt, pencil-point, conical, rounded or other 'non-cutting' or 'atraumatic' tip, and an opening in the wall of the tube, set back from the tip, which communicates with the bore of the cannula. The rounded tip parts tissue while minimising cutting or tissue damage, and injectate may be forced through the hollow bore of the cannula to exit through the opening, the injectate leaving the cannula in a direction perpendicular to the main axis of the cannula.

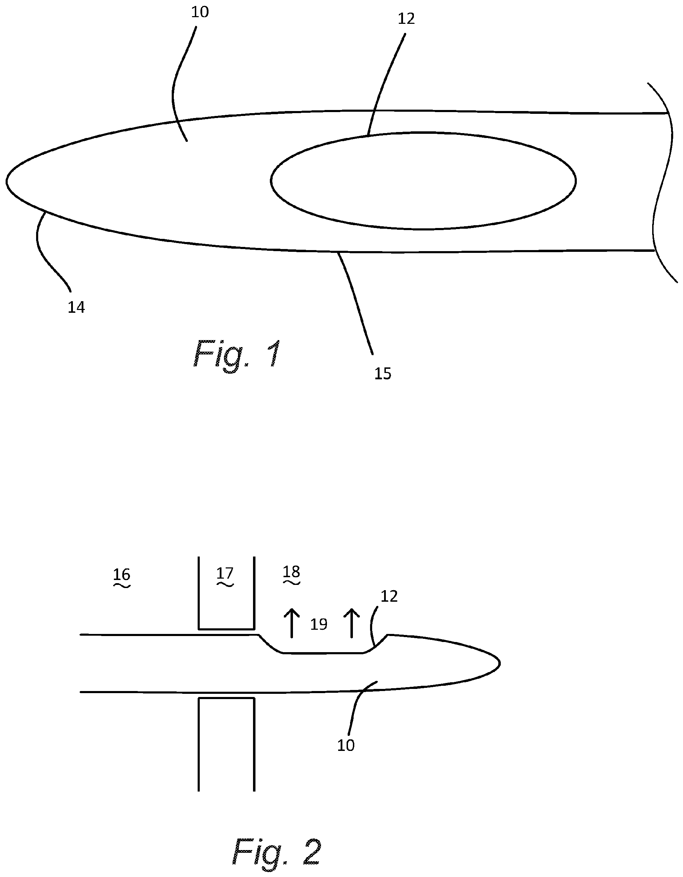

Referring to figure 1, a known design is the spinal needle 10, in which a generally oval or rectangular (symmetrical along its length) opening 12 is provided in the cylindrical wall 15 of the cannula, set back from a closed tip 14. Referring to figure 2, to administer anaesthetic solution using a spinal needle 10, the needle 10 is inserted into the body until it penetrates the dura-mater 17 and enters the subarachnoid space 18. When correctly positioned, the opening 12 is located entirely within the subarachnoid space 18, and the injectate exits the cannula into the subarachnoid space 18 as indicated by arrows 19. In this example (spinal anaesthesia), it may be determined subsequently that insufficient anaesthetic effect has been produced, leading to the need for additional anaesthesia, the risk of pain during surgery or cancellation of the planned surgical procedure.

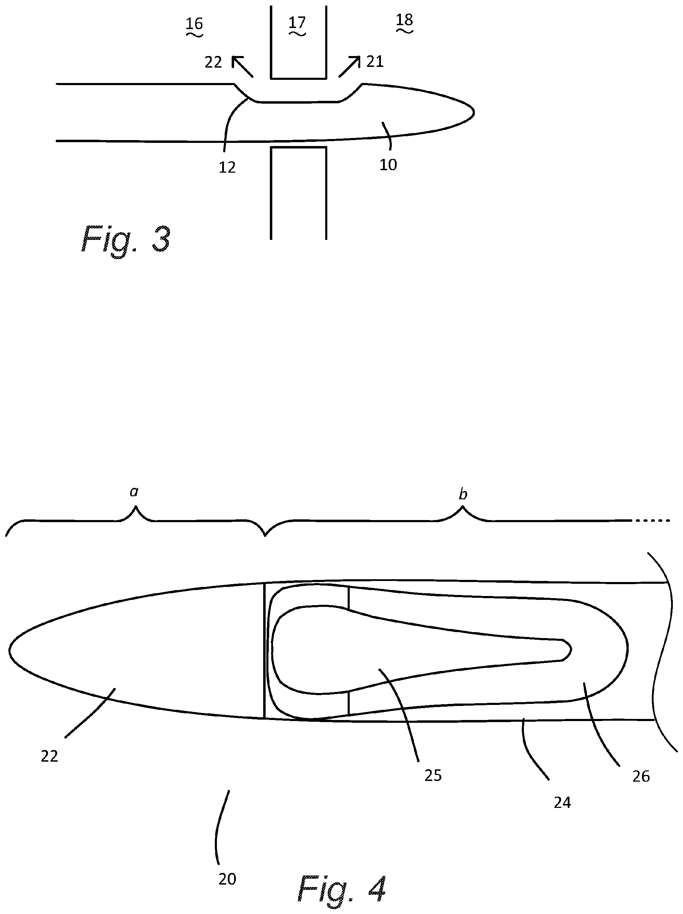

Referring to figure 3, the spinal needle 10 can however be incorrectly positioned whereby the needle 10 is not inserted sufficiently into the subarachnoid space 18, for example the straddling of the dura 17 by the opening 12. When the injectate is administered, part of the injectate exits the opening 12 into the subarachnoid space 18 as indicated by arrow 21, while part of the injectate indicated by arrow 23 exits into the epidural space 16. The injectate may also exit into the sub-dural space between layers of the dura mater. In either case, this results in less injectate being delivered to the subarachnoid space 18 than anticipated and thereby produce insufficient anaesthesia. It should be noted that there is a pressure gradient from the subarachnoid space 18 to the epidural space, which may exacerbate this problem.

US 2012/078159 A1 discloses a multi-lumen catheter with each lumen having an asymmetrical opening which widens towards the distal end. US 5 389 079 A discloses a catheter for use in spinal anaesthesia which is designed not to cut the dura matter when inserted.

The object of the present invention is to provide a cannula that increases the amount of injectate administered into the subarachnoid space in the case of incorrect placement of the needle as described above.

The term 'axial' and its cognates as used herein refers to the axis of the cannula, that is, the longitudinal axis along the cannula, unless the context requires otherwise.

According to the present invention, there is provided a cannula according to claim 1.

In this description, 'axial' and cognate terms refers to the main axis of the cannula, 'forward', 'front' and 'proximal' means towards the tip of the cannula, and 'rearward', 'back' and 'distal' means away from the tip of the cannula, unless the context requires otherwise.

The invention will now be described, by way of example, with reference to the drawings, of which
Figure 1 is a plan view of a known cannula;
Figure 2 is a side view of the known cannula in use;
Figure 3 is a further side view of the known cannula in use;
Figure 4 is a plan view of an embodiment of a cannula according to present invention;
Figure 5 is a plan view of the cannula according to present invention in more detail; and
Figure 6 is a side view of the cannula according to present invention in use.

Referring to figure 4, a cannula 20 has a tip 22 and a shaft 24, the shaft 24 having an opening 25. The tip 22 shown here tapers to a blunt, rounded end, however other tip shapes may be used as discussed below. The shaft 24 is substantially cylindrical and includes a through bore. The extent of the tip 22 is indicated by bracket *a*, while the extent of the shaft which adjoins the tip is indicated by bracket *b*.

The opening 25 extends through the shaft wall 26 so that the through bore of the cannula 20 communicates with the environment outside the cannula by the opening 25.

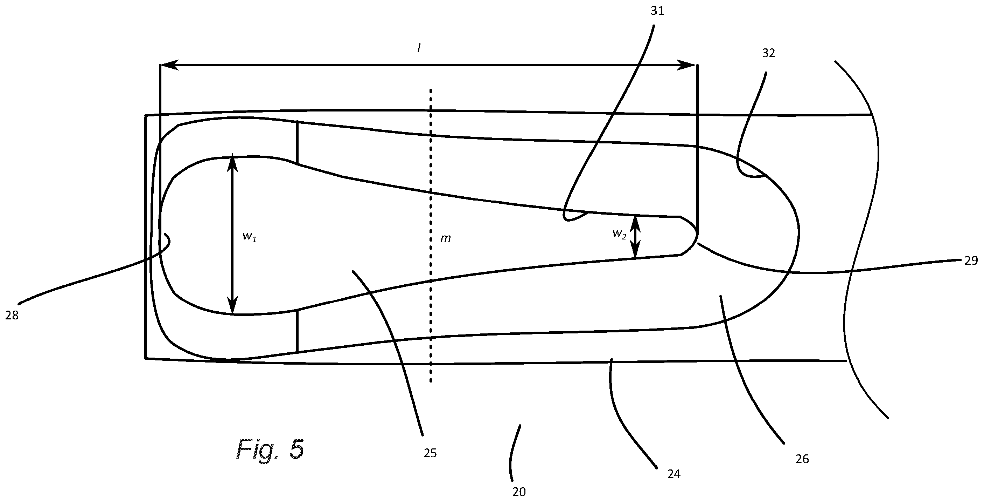

Referring to figure 5, the opening 25 is formed through the wall 26 of the shaft 24 so that the opening 25 has an inner edge 31 where the opening 25 meets the inner surface of the through bore of the cannula 20, and an outer edge 32 where the opening meets the outer surface of the shaft 24 of the cannula 20.

The opening 25 may be formed so that the shaft wall 26 exposed by the opening 25 is bevelled or sloping, so that the shape and area of the opening 25 defined by the inner edge 31 of the opening 25 has smaller dimensions than the shape and area of the opening 25 defined by the outer edge 32 of the opening 25. The inner edge 31 of the opening 25 has a front point or edge 28 proximal to the tip 22 of the cannula 20, and a rear point or edge 29 distal to the tip 22. The slope of the exposed shaft wall 26 may not be uniform, and the shaft wall 26 could be vertical (i.e. perpendicular to the centre axis of the cannula 20) or minimally inclined to the vertical in places, for example at the front point or edge 28.

The opening 25 is generally tear-drop shaped, having a maximum width *w₁* towards the front of the opening 25, and then tapering towards the rear point or edge 29. More formally, if the opening 25 is equally bisected by a midline *m*, the maximum width *w₁* will lie forward of the midline *m*. Generally, any width *w₂* that is rearward of the midline *m* will be less than the width of the opening at the midline m.

The consequence of this shape of the opening 25 is that the area of the opening rearward of the midline *m* is less than the area of the opening forward of the midline *m*.

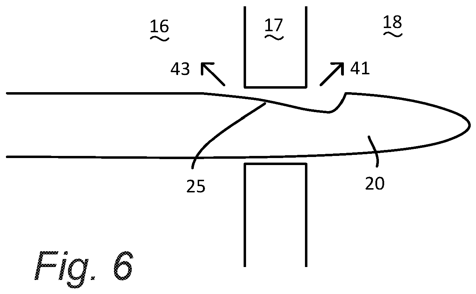

Referring to figure 6, if the cannula 20 is incorrectly positioned during a spinal anaesthetic injection so that the opening 25 straddles the dura 17, when injectate is administered through the cannula 20, the proportion of injectate exiting the opening 25 as indicated by arrow 41 into the subarachnoid space 18 is increased relative to the proportion of injectate exiting the opening 25 as indicated by arrow 43 into the epidural or sub-dural space 16, when compared to the same proportions of injectate delivered by a spinal needle or other conventional needle design.

It is desirable that the shaft wall 26 is bevelled as shown so that the inner edge 31 of the opening 25 has smaller dimensions that the outer edge 32; however, the same principle could be applied with an opening formed with no slope or bevel to the shaft wall 26, or even to an opening 25 where the slope forms an undercut so that the inner edge 31 has greater dimensions and is larger than the outer edge 32.

The midline m equally bisects the opening 25. However, an opening 25 which mitigates the problem of incorrect injectate delivery due to a membrane such as the dura-mater impinging upon the opening, could also be provided using an opening having a reduced width or a rearward tapering from a line or region which does not equally bisect the total axial length of the opening 25. The critical feature of the opening is that it axially tapers or reduces in width with axial asymmetry, so that the region at one end of the opening has a reduced area compared to the region at the opposite end.

Therefore, rather than considering a midline specifically, the opening can be characterised in that there is a dividing line is located between 30% to 70% of the way along the length of the opening, where the maximum width forward (i.e. towards the tip of the cannula) of the dividing line is greater than the maximum width rearward of the dividing line. Ideally however, the dividing line will be the midline. Having the maximum width placed in such a way allows the shape to generally narrow or taper towards the rear end of the opening, reducing the size of the opening in this region and ameliorating the effect of an incorrectly positioned cannula.

An alternative way of considering the opening is in terms of the distribution of the opening area. That is, for an opening having a dividing line is located n% of the way along the length of the opening, where n is between 30 and 70, the area of opening forward for the dividing line is greater than n% of the total opening area.

The length *l* of the opening 25 will typically 0.9mm +/- 0.3mm, though this will also typically be varied depending particularly on the inner diameter of the needle.

Spinal needles come in a range of lengths and gauges (thicknesses), to accommodate use for adults and children of varying sizes. Lengths may range from 25mm to 150mm, though 75mm to 130mm is typical; the outer diameter of the needle typical ranges from 0.5mm to 1mm, with a wall thickness of between 0.1mm and 0.5mm. The opening disclosed here may be applied to a needle of any dimension and gauge.

The width of the opening is partly determined by the width of the inner diameter of the cannula itself - this will ideally be the maximum width of the opening, as increasing the opening beyond this gives limited increase of injectate delivery and can compromise the integrity of the cannula. The width is also determined by the amount and rate of injectate to be delivered. A preferred range of the width of the opening is therefore between 20% and 100% of the width of the inner diameter of the cannula. A more preferred range of the width of the opening is between 30% and 95% of the width of the inner diameter of the cannula, and a still more preferred range is 60% to 90% of the width of the inner diameter of the cannula.

The opening is ideally located as close to the tip as possible. The length of the tip, i.e. bracket *a* figure 4, which separates the end of the needle from the frontmost edge of the 25, is typically 1mm to 2mm. The opening, as previously described is ideally set on a cylindrical shaft, but the shaft could vary in diameter, particularly towards the forward end where it meets the tip. The frontmost edge of the opening 25 is ideally set back at least 1mm, though it could be set back 1.5mm, 2mm, or further.

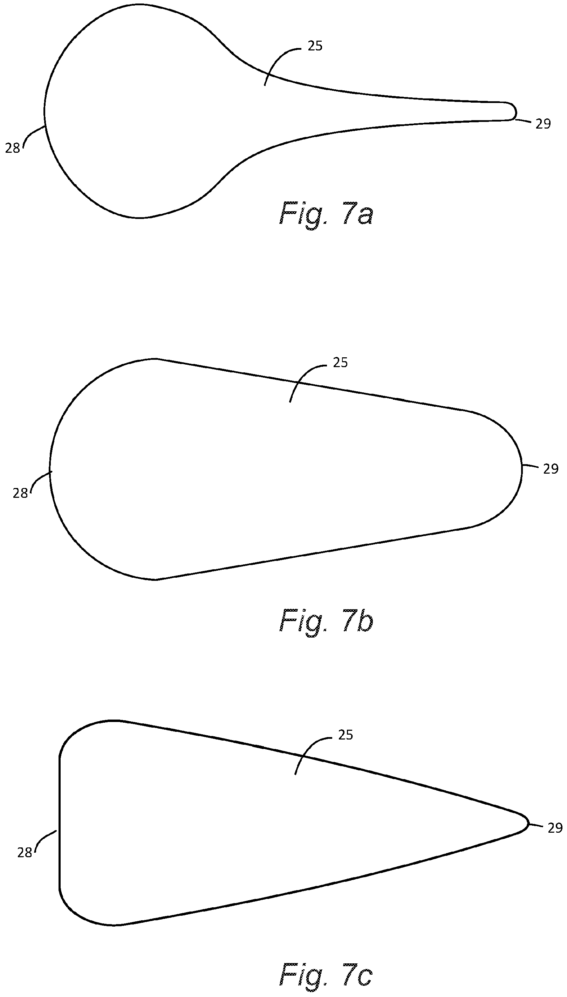

Many variations of shape are possible to benefit from the principles described herein. Referring to Figure 7a, the shape of the opening 25 could be based on a circle at the front edge 25, from which a taper leads to a small radius at the rear edge 29. Alternatively, referring to figure 7b, the radius of the lead edge 29 could be similar to that of the front edge 25, so that the taper is less pronounced. An alternative shape of the opening 25 could be based on a triangle having radiused corners, as shown in figure 7c.

The needle tip shape shown here is an example of a closed, blunt, rounded, 'pencil-point', or atraumatic type tip; however, the opening disclosed here could be applied to other cannulas, catheters and needles having other tips, for example sharp or cutting tips, cannulas having more than one internal channel, etc, and also to catheter lines that have openings at some point along their length to deliver an injectate.

The cannula will typically have a single opening for delivering injectate. However, the opening disclosed herein may be combined with other canula designs which may feature openings provided for purposes other than supplying injectate, these further openings being adapted for other purposes and not sharing the characteristics of the opening discussed herein.

As previously discussed, the cannula tip will typically be closed, and 'non-cutting' or 'atraumatic'. However the opening discussed herein could be utilised by cannulas having an open tip, and/or cannulas having a sharp or cutting tip.

The principles discussed herein could equally be applied to other medical intervention applications, with the appropriate changes in the size and position of the opening, and the material and form of the cannula. For example, an opening as disclosed herein could be formed on chest drains, which typically have a up to 34 French Gauge (11mm outer diameter - 0.445mm inner diameter), epidural catheters, which are soft plastic tubes typically 16 or 18 Gauge (1.27 - 1.65 outer diameter - 0.4-0.45 internal diameter), and other alternative anatomies for the drainage of wounds, injection into anatomical fascia, chest drains (through the pleural membrane) etc.

In this specification an apparatus/method/product "comprising" certain features is intended to be interpreted as meaning that it includes those features, but that it does not exclude the presence of other features.

Many variations are possible without departing from the scope of the present invention as defined in the appended claims.

## Claims

1. A cannula (20) for delivering injectate to human or animal tissue;
the cannula (20) having a hollow through bore; an opening in the side wall (26) of the cannula (20) adapted to allow injectate to flow from the through bore to the outside environment;
the opening (25) having a width which tapers or reduces axially in an asymmetrical manner with respect to the cannula (20)'s transverse axis, so that the region at the rear end of the opening (25) has a reduced area compared to the region at the opposite end, **characterised in that** only one opening (25) is provided.

2. A cannula (20) according to either previous claim, wherein the cannula (20) includes a tip (22), and the width of the opening (25) tapers or reduces distally from the tip (22).

3. A cannula (20) according to claim 3, wherein the tip (22) of the cannula (20) is closed.

4. A cannula (20) according to any previous claim, wherein the maximum width of the opening (25) is forward from a bisecting midline (m) of the opening (25).

5. A cannula (20) according to any previous claim, wherein the width of the opening (25) decreases from the maximum width of the opening (25) to the rear point or end of the opening (25).

6. A cannula (20) according to any previous claim, wherein for a dividing line located between 30% to 70% of the way along the length of the opening (25), the maximum width forward of the dividing line is greater than the maximum width rearward of the dividing line.

7. A cannula (20) according to any previous claim, wherein for a dividing line located n% of the way along the length of the opening (25), where n is between 30 and 70, the area of opening (25) forward for the dividing line is greater than n% of the total opening (25) area.

## Patentansprüche

1. Kanüle (20) für das Zuführen eines Injektats in ein menschliches oder tierisches Gewebe;
Die Kanüle (20) weist eine hohle Durchgangsbohrung auf;
eine Öffnung an der Seitenwand (26) der Kanüle (20), die so angepasst ist, dass sie es dem Injektat ermöglicht, von der Durchgangsbohrung nach außen zu fließen;
die Öffnung (25) weist eine Breite auf, die konisch zuläuft oder sich axial in asymmetrischer Weise in Bezug auf die Querachse der Kanüle (20) reduziert, so dass der Bereich am hinteren Ende der Öffnung (25) eine reduzierte Fläche im Vergleich zu dem Bereich am entgegengesetzten Ende aufweist, **dadurch gekennzeichnet, dass** nur eine Öffnung (25) bereitgestellt wird.

2. Kanüle (20) nach einem der vorstehenden Ansprüche, wobei die Kanüle (20) eine Spitze (22) einschließt, und die Breite der Öffnung (25) konisch zuläuft oder sich distal von der Spitze (22) reduziert.

3. Kanüle (20) nach Anspruch 3, wobei die Spitze (22) der Kanüle (20) geschlossen ist.

4. Kanüle (20) nach einem der vorstehenden Ansprüche, wobei die maximale Breite der Öffnung (25) vor einer winkelhalbierenden Mittellinie (m) der Öffnung (25) liegt.

5. Kanüle (20) nach einem der vorstehenden Ansprüche, wobei die Breite der Öffnung (25) von der maximalen Breite der Öffnung (25) zu dem rückseitigen Punkt oder Ende der Öffnung (25) abnimmt.

6. Kanüle (20) nach einem der vorstehenden Ansprüche, wobei für eine Teilungslinie, die zwischen 30% und 70% des Weges entlang der Länge der Öffnung (25) liegt, die maximale Breite vor der Teilungslinie größer ist als die maximale Breite hinter der Teilungslinie.

7. Kanüle (20) nach einem der vorstehenden Ansprüche, wobei für eine Teilungslinie, die auf n% des Weges entlang der Länge der Öffnung (25) liegt, wobei n zwischen 30 und 70 liegt, die Fläche der Öffnung (25) vor der Teilungslinie größer ist als n% der Fläche der Gesamtöffnung (25).

## Revendications

1. Canule (20) destinée à administrer un produit injectable à un tissu humain ou animal ;
la canule (20) présentant un alésage traversant creux ;
une ouverture dans la paroi latérale (26) de la canule (20) conçue pour permettre à la solution injectable de s'écouler de l'alésage traversant vers l'environnement extérieur ;
l'ouverture (25) présentant une largeur qui s'effile ou se rétrécit axialement de manière asymétrique par rapport à l'axe transversal de la canule (20), de manière à ce que la zone située à l'extrémité arrière de l'ouverture (25) présente une surface réduite par rapport à la zone située à l'extrémité opposée, **caractérisée en ce qu'**une seule ouverture (25) est prévue.

2. Canule (20) selon une quelconque revendication précédente, dans laquelle la canule (20) comporte une pointe (22), et la largeur de l'ouverture (25) s'effile ou se réduit de manière distale à partir de la pointe (22).

3. Canule (20) selon la revendication 3, dans laquelle la pointe (22) de la canule (20) est fermée.

4. Canule (20) selon une quelconque revendication précédente, dans laquelle la largeur maximale de l'ouverture (25) est en avant d'une ligne médiane bissectrice (m) de l'ouverture (25).

5. Canule (20) selon une quelconque revendication précédente, dans laquelle la largeur de l'ouverture (25) décroit depuis la largeur maximale de l'ouverture (25) jusqu'au point arrière ou à l'extrémité arrière de l'ouverture (25).

6. Canule (20) selon une quelconque revendication précédente, dans laquelle, pour une ligne de séparation située entre 30 % et 70 % du chemin le long de la longueur de l'ouverture (25), la largeur maximale en avant de la ligne de séparation est supérieure à la largeur maximale en arrière de la ligne de séparation.

7. Canule (20) selon une quelconque revendication précédente, dans laquelle, pour une ligne de séparation située à n % du chemin le long de la longueur de l'ouverture (25), où n est compris entre 30 et 70, la surface de l'ouverture (25) en avant pour la ligne de séparation est supérieure à n % de la surface totale de l'ouverture (25).
